# EUROPEAN PATENT APPLICATION

(11) **EP 2 345 382 A1**
(43) Date of publication of application: **20.07.2011**
(21) Application number: 11150757.0
(22) Date of filing: 12.01.2011
(51) Int. Cl.: A61B 17/70

(54) **Articulating interspinous process clamp**

(30) Priority: 13.01.2010 US 687046
(71) Applicant: Kyphon SÀRL, 2000 Neuchâtel (CH)
(72) Inventor: Marik, Greg C, Collierville, TN 38017 (US); Metcalf, Newton H Jr., Memphis, TN 38111 (US)
(74) Representative: Price, Nigel John King

(57) **Abstract**

Medical devices for the treatment of spinal conditions are described herein. Such a medical device (100) may include a pair of plates (20) with spikes (30) adapted to be embedded in a superior spinous process and an adjacent inferior spinous process. The device may also allow relative motion between adjacent spinous processes.

## Description

### Background

This invention relates generally to the treatment of spinal conditions, and more particularly, to the treatment of spinal stenosis using devices for implantation between adjacent spinous processes.

The clinical syndrome of neurogenic intermittent claudication due to lumbar spinal stenosis is a frequent source of pain in the lower back and extremities, leading to impaired walking, and causing other forms of disability in the elderly. Although the incidence and prevalence of symptomatic lumbar spinal stenosis have not been established, this condition is the most frequent indication of spinal surgery in patients older than 65 years of age.

Lumbar spinal stenosis is a condition of the spine characterized by a narrowing of the lumbar spinal canal. With spinal stenosis, the spinal canal narrows and pinches the spinal cord and nerves, causing pain in the back and legs. It is estimated that approximately 5 in 10,000 people develop lumbar spinal stenosis each year. For patients who seek the aid of a physician for back pain, approximately 12% - 15% are diagnosed as having lumbar spinal stenosis.

Common treatments for lumbar spinal stenosis include physical therapy (including changes in posture), medication, and occasionally surgery. Changes in posture and physical therapy may be effective in flexing the spine to decompress and enlarge the space available to the spinal cord and nerves - thus relieving pressure on pinched nerves. Medications such as NSAIDS and other antiinflammatory medications are often used to alleviate pain, although they are not typically effective at addressing spinal compression, which is the cause of the pain.

Surgical treatments are more aggressive than medication or physical therapy, and in appropriate cases surgery may be the best way to achieve lessening of the symptoms of lumbar spinal stenosis. The principal goal of surgery is to decompress the central spinal canal and the neural foramina, creating more space and eliminating pressure on the spinal nerve roots. The most common surgery for treatment of lumbar spinal stenosis is direct decompression via a laminectomy and partial facetectomy. In this procedure, the patient is given a general anesthesia as an incision is made in the patient to access the spine. The lamina of one or more vertebrae is removed to create more space for the nerves. The intervertebral disc may also be removed, and the adjacent vertebrae may be fused to strengthen the unstable segments. The success rate of decompressive laminectomy has been reported to be in excess of 65%. A significant reduction of the symptoms of lumbar spinal stenosis is also achieved in many of these cases.

Alternatively, the vertebrae can be distracted and an interspinous process device implanted between adjacent spinous processes of the vertebrae to maintain the desired separation between the vertebral segments. Such interspinous process devices typically work for their intended purposes, but some could be improved. For example, some current devices can migrate due to the constant bending and twisting of the spine. In addition, some other current devices that are less prone to migration restrict the range of motion for the patient.

Thus, a need exists for improvements in interspinous process devices.

### Summary

The interspinous process device described herein includes a pair of plates, with each plate of the pair adapted to be located on opposite lateral sides of adjacent spinous processes, and a plurality of spikes located along the interior faces of at least an inferior or superior portion of each of the plates. The interspinous process device also may include a hinge along a medial portion of the plates to allow pivoting motion of the adjacent spinous process when the spine moves in flexion.

The interspinous process device of this invention is implanted such that one plate is located along one lateral side of a superior spinous process and an adjacent inferior spinous process with the other plate located along the opposite lateral side of the superior spinous process and the adjacent inferior spinous process. The spikes located along an interior surface of the plates are embedded in the spinous processes. In one embodiment, the spikes are located along both the superior and inferior interior portions of both plates. Embedding spikes in both the superior and inferior spinous processes prevents migration of the device and prevents compression of the adjacent vertebrae since the spikes and plates, which are substantially rigid, hold the adjacent spinous processes apart. In another embodiment, the spikes are located along only either the interior part of the superior portion of both plates or the interior part of the inferior portion of both plates. With this second embodiment, the adjacent spinous processes have some freedom of movement with respect to each other. A bumper may be located between the plates to limit spinal extension and prevent compression of the adjacent vertebrae and thus ensure adequate pain relief for the patient. The spikes also prevent migration of the device.

### Brief Description of the Drawings

FIG. 1 is a rear perspective view of an interspinous process device and a portion of a spine on which it is located;

FIG. 2 is a perspective, exploded view of an interspinous process device;

FIG. 3 is a perspective, exploded view of another embodiment of an interspinous process device;

FIG. 4 is a rear elevation view of an interspinous process device and a portion of a spine on which it is located;

FIG. 5 is a side elevation view of an interspinous process device and a portion of a spine on which it is located;

FIG. 6 is a rear perspective view of another embodiment of an interspinous process device and a portion of a spine on which it is located;

FIG. 7 is a perspective, exploded view of an interspinous process device shown in FIG. 6;

FIG. 8 is a rear elevation view of an interspinous process device shown in FIG. 6 and a portion of a spine on which it is located; and

FIG. 9 is a side elevation view of an interspinous process device shown in FIG. 6 and a portion of a spine on which it is located.

### Detailed Description

As used in this specification and the appended claims, the singular forms "a," "an" and "the" include plural referents unless the context clearly dictates otherwise. Thus, for example, the term "a member" is intended to mean a single member or a combination of members, and "a material" is intended to mean one or more materials, or a combination thereof. Furthermore, the words "proximal" and "distal" refer to directions closer to and away from, respectively, an operator (e.g., surgeon, physician, nurse, technician, etc.) who would insert the medical device into the patient, with the tip-end (i.e., distal end) of the device inserted inside a patient's body first. Thus, for example, the device end first inserted inside the patient's body would be the distal end of the device, while the device end last to enter the patient's body would be the proximal end of the device.

As used in this specification and the appended claims, the term "body" when used in connection with the location where the device is to be placed to treat lumbar spinal stenosis, or to teach or practice implantation methods for the device, means a mammalian body. For example, a body can be a patient's body, or a cadaver, or a portion of a patient's body or a portion of a cadaver.

As used in this specification and the appended claims, the term "parallel" describes a relationship, given normal manufacturing or measurement or similar tolerances, between two geometric constructions (e.g., two lines, two planes, a line and a plane, two curved surfaces, a line and a curved surface or the like) in which the two geometric constructions are substantially non-intersecting as they extend substantially to infinity. For example, as used herein, a line is said to be parallel to a curved surface when the line and the curved surface do not intersect as they extend to infinity. Similarly, when a planar surface (i.e., a two-dimensional surface) is said to be parallel to a line, every point along the line is spaced apart from the nearest portion of the surface by a substantially equal distance. Two geometric constructions are described herein as being "parallel" or "substantially parallel" to each other when they are nominally parallel to each other, such as for example, when they are parallel to each other within a tolerance. Such tolerances can include, for example, manufacturing tolerances, measurement tolerances or the like.

As used in this specification and the appended claims, the terms "normal", perpendicular" and "orthogonal" describe a relationship between two geometric constructions (e.g., two lines, two planes, a line and a plane, two curved surfaces, a line and a curved surface or the like) in which the two geometric constructions intersect at an angle of approximately 90 degrees within at least one plane. For example, as used herein, a line is said to be normal, perpendicular or orthogonal to a curved surface when the line and the curved surface intersect at an angle of approximately 90 degrees within a plane. Two geometric constructions are described herein as being "normal", "perpendicular", "orthogonal" or "substantially normal", "substantially perpendicular", "substantially orthogonal" to each other when they are nominally 90 degrees to each other, such as for example, when they are 90 degrees to each other within a tolerance. Such tolerances can include, for example, manufacturing tolerances, measurement tolerances or the like.

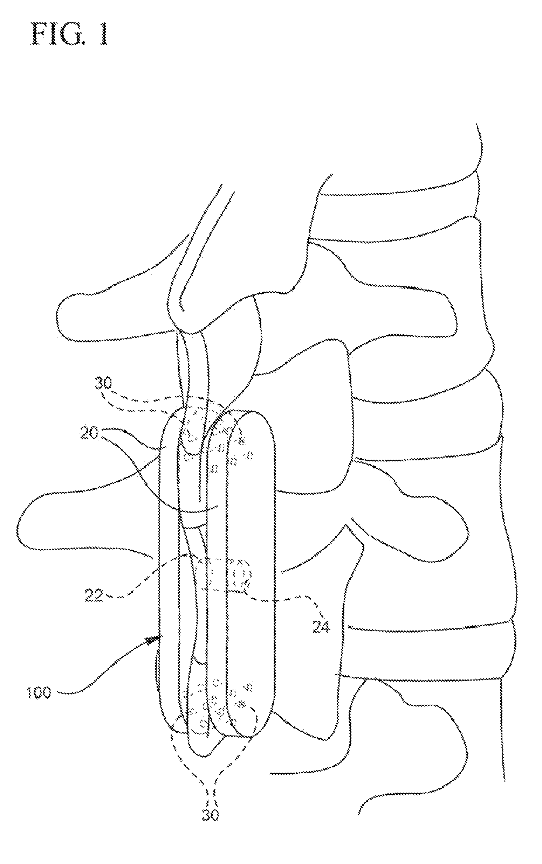

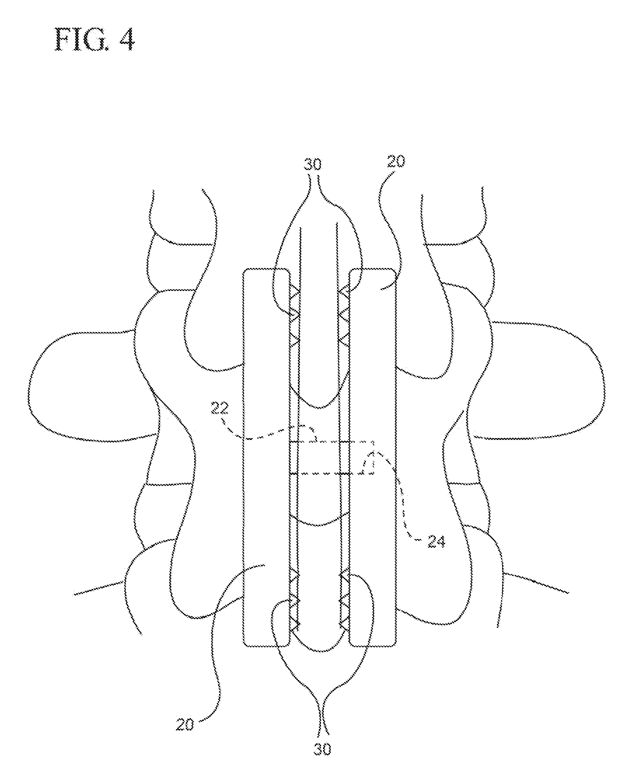

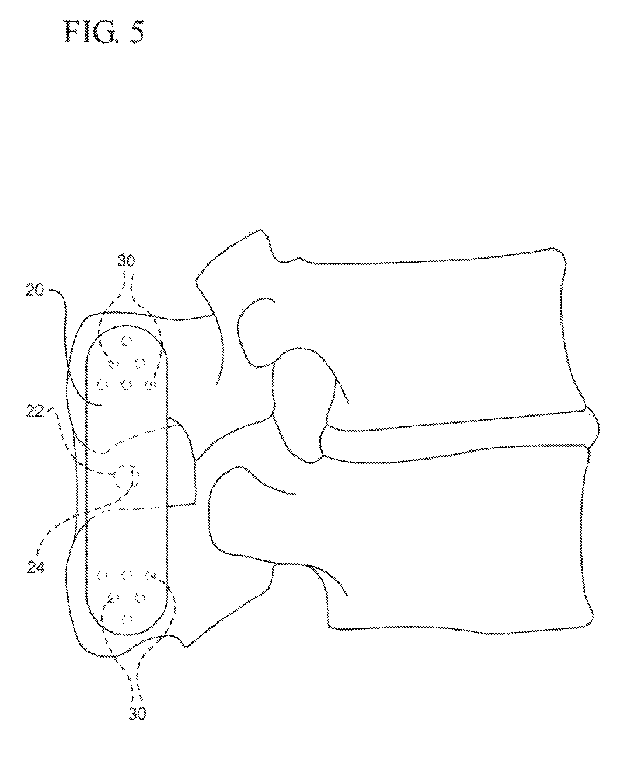

FIGS. 1 - 5 illustrate an embodiment of an interspinous process device 100. Device 100 includes a pair of plates 20, with each plate 20 of the pair adapted to be located on one lateral side of adjacent spinous processes, and a plurality of spikes 30 located along the interior faces of at least an inferior portion or superior portion of each of plates 20. A post 22 extends from one of plates 20. The other plate 20 defines a cut-out portion 24 for receiving an end portion of post 22. Preferably, cut-out portion 24 has a diameter slightly smaller than the diameter of the end portion of post 22 to provide an interference fit between those two elements. This ensures that plates 20 stay connected to each other via post 22. Of course, alternative mechanisms could be used rather than having cut-out portion 24 engage the end portion of post 22 in an interference fit. For example, the end portion of post 22 could be threaded and cut-out portion 24 could have complementary threads to allow post 22 to be screwed into place.

Spikes 30 are designed to become embedded in the lateral surface of the spinous processes when plates 20 are squeezed laterally toward each other during implantation. Each spike 30 has a pointed or otherwise sharp tip to facilitate the engagement of each spike 30 with the bone of the spinous process into which that spike is to be embedded. With spikes 30 along the superior portion of each of plates 20 embedded in the lateral surfaces of both sides of a superior spinous process and spikes 30 along the inferior portion of each of plates 20 embedded in the lateral surfaces of both sides of an adjacent inferior spinous process, plates 20, which are substantially rigid, maintain the spacing between the adjacent vertebrae. A physician can distract the adjacent vertebrae to decompress and relieve pressure on pinched nerves and then implant device 100 to maintain the desired spacing and distraction for the patient. Thus, embedding spikes 30 in both the superior and inferior spinous processes prevents migration of device 100 and prevents compression between the adjacent vertebrae.

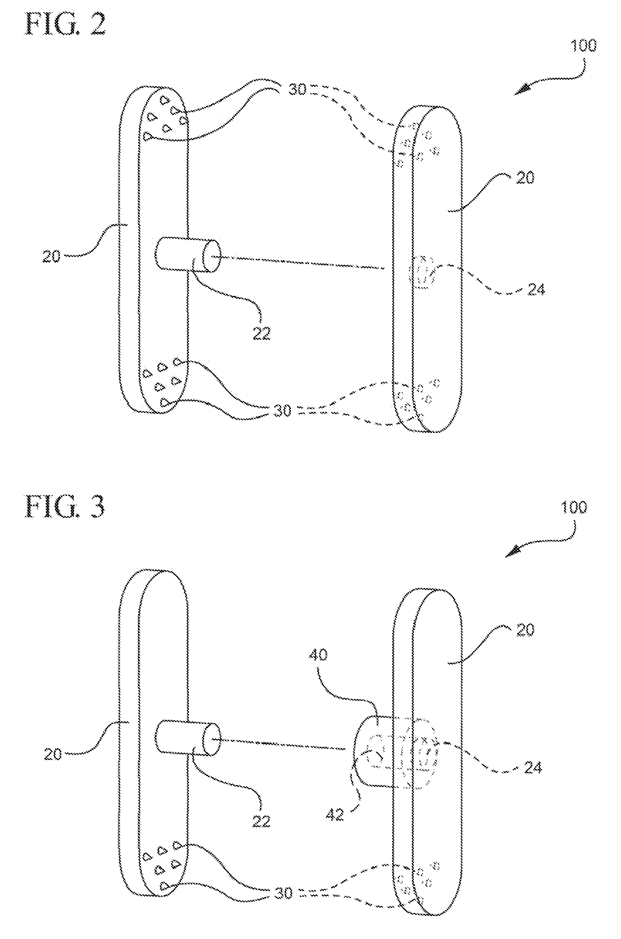

FIG. 3 illustrates a variation of interspinous process device 100. In this variation, spikes 30 are included only on the inferior interior portion of each of plates 20, thus allowing the superior spinous process to move with respect to device 100 when spikes 30 are embedded in inferior spinous process. Alternatively, spikes 30 could be located only on the superior interior portions of each of plates 20, thus allowing the inferior spinous process to move with respect to device 100 when spikes 30 are embedded in the superior spinous process. By locating spikes 30 only along the superior portion or the inferior portion of plates 20, the adjacent spinous processes may articulate with respect to each other allowing greater freedom of movement for the patient. In addition, spikes 30 still affix device 100 in place to prevent migration of device 100.

As shown in FIG. 3, a bumper 40 may be disposed between plates 20 to act as an extension stop for the adjacent spinous processes to which device 100 is affixed since, in this embodiment, either the superior spinous process or the inferior spinous process is allowed to move with respect to device 100 and thus plates 20 and spikes 30 would not maintain the space between adjacent vertebrae. Bumper 40 and one plate 20 are preferably formed as a single unit. Bumper 40 defines an opening 42 therein, which allows bumper 40 to fit over post 22. The opening may have a diameter slightly smaller than the diameter of the portion of post 22 that is adapted to fit within the opening. There would thus be an interference fit between post 22 and opening 42 of bumper 40 to hold plates 20 together as a single unit. Of course, cut out portion 24 could be used to engage the end of post 22 as in the previous embodiment. Alternatively, some other mechanism such as complementary threads could be used to connect post 22 within the opening of bumper 40.

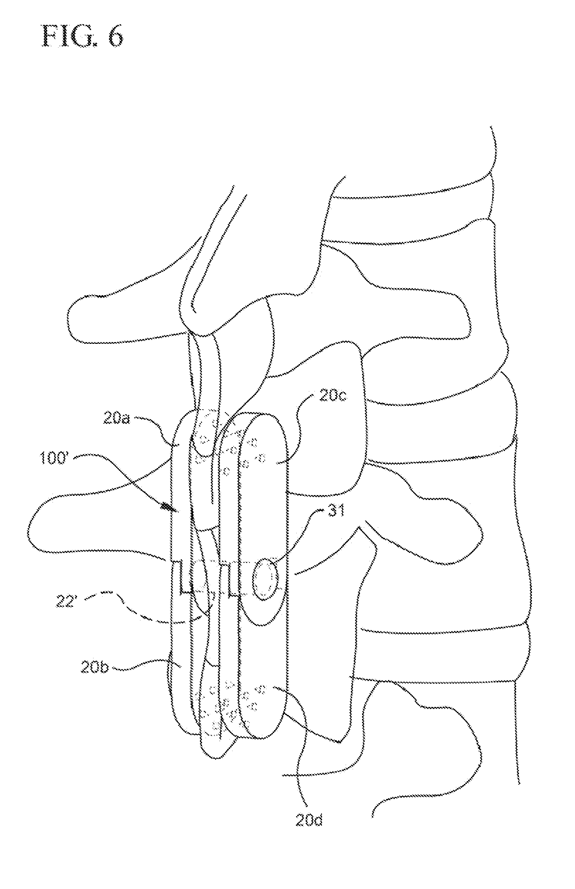

FIGS. 6 ― 9 disclose another embodiment of an interspinous process device. In the embodiment shown in these FIGS., device 100' comprises a first superior plate 20a, a first inferior plate 20b, a second superior plate 20c and a second inferior plate 20d. The inferior portion of first superior plate 20a includes a first inferior flange 21 a and the superior portion of first inferior plate 20b includes a first superior flange 21 b. First inferior flange 21 a defines a first superior opening 26a therein. A first superior end wall 23a defines the superior end of first inferior flange 21 a. Post 22' extends from first superior flange 21 b. A first inferior end wall 23b defines the inferior end of first superior flange 21 b. The inferior portion of second superior plate 20c includes a second inferior flange 21 c and the superior portion of second inferior plate 20d includes a second superior flange 21 d. A second superior end wall 23c defines the superior end of second inferior flange 21 c. A second inferior end wall 23d defines the inferior end of second superior flange 21d. Second inferior flange 21 c defines a second superior opening 26c therein. Second superior flange 21 d defines a first inferior opening 26d therein.

First superior plate 20a is connected to first inferior plate 20b so that first inferior flange 21 a and first superior flange 21 b abut and overlap to form a first half lap joint 25 to allow first superior plate 20a to move with respect to first inferior plate 20b. In this orientation, post 22' extends through first superior opening 26a. The diameter of first superior opening 26a should be slightly larger than the diameter of post 22' to allow first superior plate 20a to pivot or otherwise move with respect to first inferior plate 20b. In addition, first superior end wall 23a, first inferior end wall 23b, the inferior end of first inferior flange 21 a, and the superior end of first superior flange 21 b are preferably curved to allow relative pivoting motion between first superior plate 20a and first inferior plate 20b. Second superior plate 20c is connected to second inferior plate 20d along second inferior flange 21 c and second superior flange 21 d, which abut and overlap to form a second half lap joint 27 to allow second superior plate 20c to move with respect to second inferior plate 20d. The diameter of second superior opening 26c should be slightly larger than the diameter of post 22' to allow second superior plate 20c to pivot with respect to first inferior plate 20b and second inferior plate 20d. The diameter of first inferior opening 26d may be slightly smaller than the diameter of the end portion of post 22' so that the end portion of post 22' fits in first inferior opening 26d in an interference fit. Alternatively, second inferior plate 20d could be otherwise fixed to post 22'. This arrangement allows first inferior plate 20b and second inferior plate 20d to be substantially fixed with respect to each other yet allow first superior plate 20a to pivot with respect to first inferior plate 20b and second inferior plate 20d. Similarly, second superior plate 20c is thus allowed to pivot with respect to first inferior plate 20b and second inferior plate 20d. Preferably, a hub 31 fits over the end of post 22' in an interference fit, or is otherwise fixed to post 22', to hold first superior plate 20a, first inferior plate 20b, second superior plate 20c and second inferior plate 20d together so they all operate as a single unit.

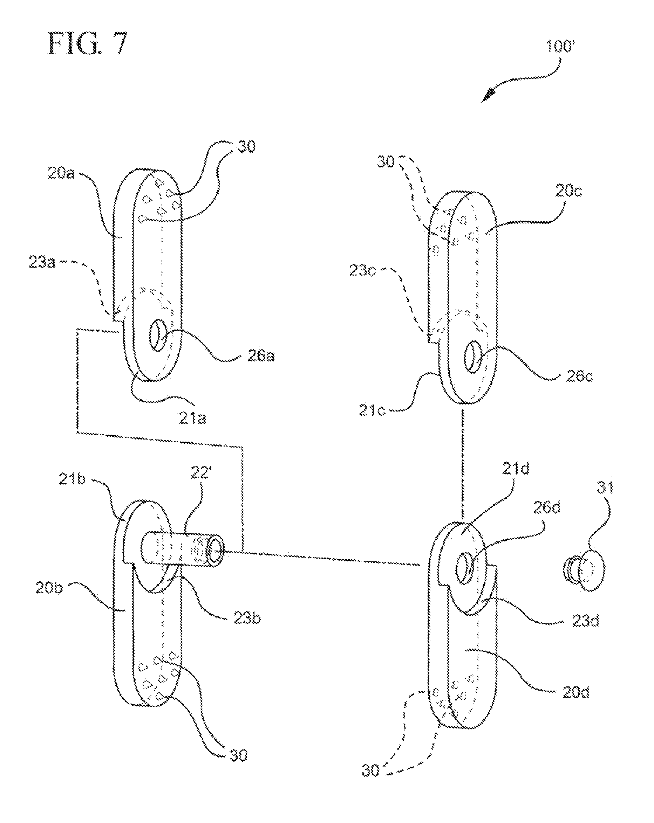

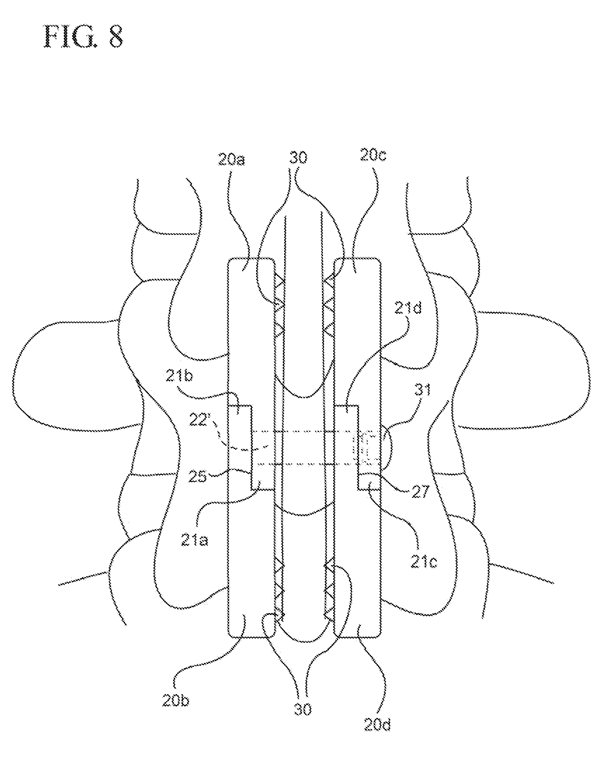

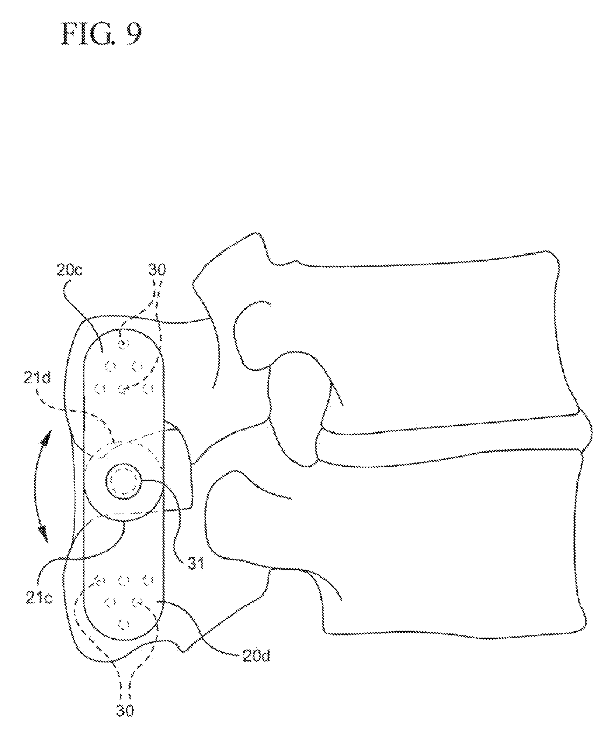

As discussed in connection with the embodiment shown in FIG. 2, spikes 30 are adapted to be embedded in adjacent spinous processes to maintain distraction between adjacent vertebrae and provide pain relief for the patient. However, even though device 100' is fixed to adjacent vertebrae, lap joints 25 and 27 allow both superior plates 20a, 20c to move with respect to both inferior plates 20b, 20d thus allowing more normal biomechanical movement between the adjacent vertebrae. This ensures greater freedom of movement for the patient. Although a lap joint is shown in the embodiment of FIGS. 6 - 9, it is to be understood that other joints, such as a ball joint, could be used to allow the superior plates to move with respect to the inferior plates.

Device 100 and 100' can be constructed with various biocompatible materials such as, for example, titanium, titanium alloy, surgical steel, biocompatible metal alloys, stainless steel, Nitinol, plastic, polyetheretherketone (PEEK), carbon fiber, ultra-high molecular weight (UHMW) polyethylene, and other biocompatible polymeric materials. The material of device 100 and 100' can have, for example, a compressive strength similar to or higher than that of bone. Alternatively, device 100 and 100' may have a lower elastic modulus than bone.

While various embodiments of an interspinous process device have been described above, it should be understood that they have been presented by way of example only, and not limitation. Many modifications and variations will be apparent to the practitioner skilled in the art. The foregoing description of an interspinous process device is not intended to be exhaustive or to limit the scope of the claimed invention. It is intended that the scope of the invention be defined by the following claims and their equivalents.

## Claims

1. A device, comprising:
a first lateral plate having a superior interior portion and an inferior interior portion;
a second lateral plate having a superior interior portion and an inferior interior portion connectable to the first lateral plate;
a plurality of spikes located along the superior interior portion of the first lateral plate; and
a plurality of spikes located along the superior interior portion of the second lateral plate.

2. The device of claim 1 further comprising a bumper located between the first lateral plate and the second lateral plate.

3. The device of claim 2 wherein the bumper is affixed to a medial interior portion of one of the first lateral plate and the second lateral plate.

4. A device, comprising:
a first lateral plate having a superior interior portion and an inferior interior portion;
a second lateral plate having a superior interior portion and an inferior interior portion connectable to the first lateral plate;
a plurality of spikes located along the inferior interior portion of the first lateral plate; and
a plurality of spikes located along the inferior interior portion of the second lateral plate.

5. The device of claim 4 further comprising a bumper located between the first lateral plate and the second lateral plate.

6. The device of claim 5 wherein the bumper is affixed to a medial interior portion of one of the first lateral plate and the second lateral plate.

7. A device, comprising:
a first superior plate;
a first inferior plate pivotally connected to the first superior plate;
a second superior plate;
a second inferior plate pivotally connected to the second superior plate; and
a plurality of spikes located along an interior portion of the first superior plate, an interior portion of the first interior plate, an interior portion of the second superior plate, and an interior portion of the second inferior plate.

8. The device of claim 7 wherein the first superior plate is movable with respect to the first inferior plate and the second inferior plate and is fixed with respect to the second superior plate and the second superior plate is movable with respect to the first inferior plate and the second inferior plate and is fixed with respect to the first superior plate

9. The device of claim 7 wherein the first superior plate is connected to the first inferior plate by a first lap joint and the second superior plate is connected to the second inferior plate by a second lap joint.

10. The device of claim 7 wherein the first superior plate is connected to the first inferior plate by a first ball joint and the second superior plate is connected to the second inferior plate by a second ball joint.
